# EUROPEAN PATENT SPECIFICATION

(11) **EP 0 637 633 B1**
(45) Date of publication and mention of the grant of the patent: **18.07.2001**
(21) Application number: 94112272.3
(22) Date of filing: 05.08.1994
(51) Int. Cl.: C12Q 1/56

(54) **Method of determining activity of tested substance**
Verfahren zur Bestimmung der Wirksamkeit einer Testsubstanz
Méthode pour la détermination de l'activité d'une substance d'essai

(30) Priority: 06.08.1993 JP 21517793
(43) Date of publication of application: 08.02.1995
(73) Proprietor: NIPPON ZOKI PHARMACEUTICAL CO. LTD., Osaka (JP)
(72) Inventor: Nishikawa, Katsumi, c/o Inst.of Bio-Active Science, Yashiro-cho, Katoh-gun,Hyogo (JP); Ishibashi, Nobuyuki, c/o Ono Greenery Factory, Furukawa-cho, Ono-shi, Hyogo (JP)
(74) Representative: Hansen, Bernd, Dr. Dipl.-Chem.

(56) References cited:
- EP-A- 0 078 764
- EP-A- 0 190 766
- EP-A- 0 259 857
- WO-A-91/17258
- US-A- 4 882 272
- H. U. BERGMEYER (EDITOR-IN-CHIEF): "Methods of Enzymatic Analysis, Vol. V." 1984 , VERLAG CHEMIE GMBH. , WEINHEIM, DE XP002046938 014041 * page 316 - page 351 * * page 394 - page 399 *

## Description

### Detailed Description of the Invention:

The present invention relates to a method of measuring the activity of a tested substance to the production of a blood coagulation factor XII in an active form and, more particularly, it relates to a method of measuring the activity of a substance which inhibits or promotes the production of the blood coagulation factor XII.

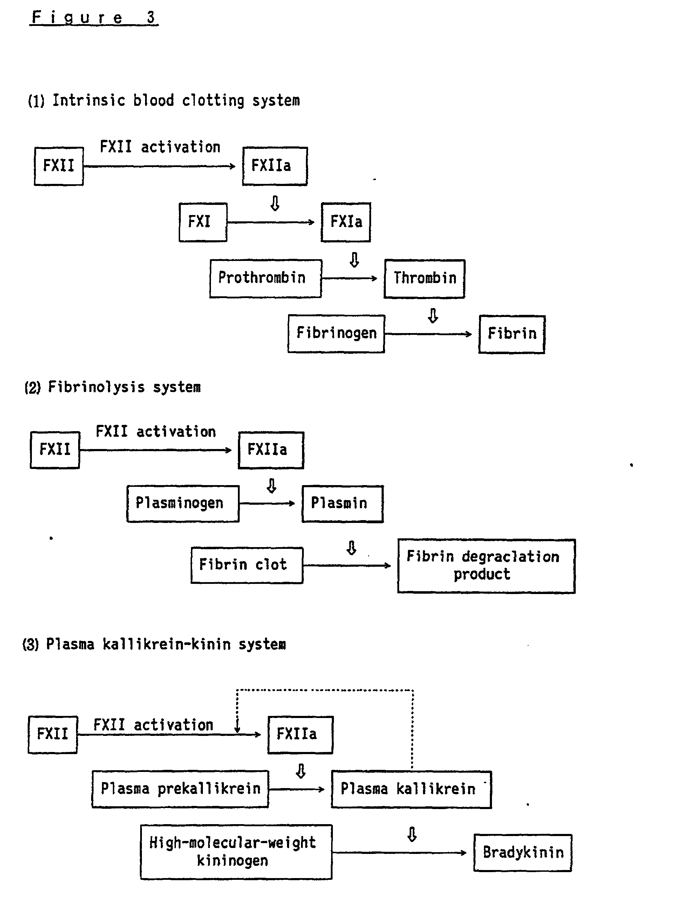

The blood coagulation factor XII (hereinafter abbreviated as an FXII) which is called a Hageman factor is a factor which is activated for the first time in the chain reaction of intrinsic blood clotting. An activated blood coagulation factor XII (the blood coagulation factor XII in an active form; hereinafter abbreviated as an FXIIa) activates the blood coagulation factor XII, then a series of the reaction system of intrinsic blood clotting as shown in (1) of Fig. 3 proceeds and, finally, an insoluble fibrin is produced whereupon the intrinsic blood clotting reaction is accomplished.

A series of the mechanisms for dissolving the fibrin clot is a fibrinolysis system and it has been known that FXIIa participates in the initial phase of the fibrinolysis system. Thus, as shown in (2) of Fig. 3, plasmin which plays a main role in the fibrinolysis is present in plasma as an insoluble plasminogen but FXIIa is one of the activators which activate plasminogen. The reaction by which the fibrin clot is produced by coagulation of plasmin is the final stage of the fibrinolysis system.

In addition, activation of FXII is an initial stage of the plasma kallikrein-kinin system. In the plasma kallik-rein-kinin system, it is believed that a series of enzymatic reaction systems are involved as a result of the activation of FXII by injury, invaded stimulation, etc. to the tissues in vivo. Thus, as shown in (3) of Fig. 3, the activated FXIIa acts on the plasma prekallikrein which is also present in the plasma, converts it to a plasma kallikrein which is an enzyme in an active form and then the plasma kallikrein acts on the high-molecular-weight kininogen (hereinafter abbreviated as an HK) to liberate bradykinin.

EP-A-0 259 857 discloses a method for assaying a physiologically active substance (a drug), which comprises (i) activating F-XII in animal plasma in the presence of a drug to be tested in order to convert plasma prekallikrein into kallikrein and (ii) measuring the amount of kallikrein produced by activated F-XII.

EP-A-0 078 764 discloses a method for determining quantitatively the amount of F-XII in human plasma, which comprises (i) adding an activator to the plasma sample in order to activate F-XII, and (ii) reacting activated F-XII with a tripeptide derivative in order to produce a (fluorophore or chromophore) product, which can be measured spectrophotometrically.

Kinins such as bradykinin which are the products in the kallikrein-kinin system exhibit various physiological activities such as decreasing the blood pressure due to dilation of peripheral blood vessels,promotion in permeability of blood vessels, contraction or relaxation of smooth muscle, induction of pain , induction of inflammation, migration of leucocytes, libreration of catecholamine from the adrenal cortex, etc. and are also known as mediators in acute inflammation including allergic reactions whereby their existence in vivo has a deep significance.

As such, FXIIa is a very important factor in the initial stage of the intrinsic blood clotting system, fibrinolysis system and plasma kallikrein-kinin sytem. Accordingly, the substances affecting the production of FXIIa are expected to be useful as drugs in the areas of blood clotting and fibrinolysis and, in addition, there is a possibility that they can be used as antiinflammatory, analgesic and antiallergic drugs since bradykinin liberated in the plasma kallikrein-kinin system has various physiological activities as mentioned above.

Accordingly, the establishment of a method wherein the action and activity of a substance which inhibits or promotes the production of FXIIa which is an initiation factor of the intrinsic blood clotting system, fibrinolysis system and plasma kallikrein-kinin system can be measured in a simple, convenient, prompt and precise manner would be a very effective means for determining the action helpful in adjusting the bioregulations as mentioned above and also for developing such drugs.

An object of the present invention is to offer an in vitro method of measuring the activity of a tested substance which inhibits or promotes the production of FXIIa participating in various physiological reaction systems in vivo in a simple, convenient, prompt and precise manner.

Thus, the method of measuring the activity of a tested substance in accordance with the present invention is characterized in that an activation reaction of a blood coagulation factor XII is initiated by adding a blood coagulation factor XII activator to the animal plasma in the presence of the tested substance, then the reaction is stopped and the blood coagulation factor XII in an active form produced in the above reaction is quantitatively determined.

With respect to the plamsa of animals which can be used in the measuring method of the present invention, the plasma of any animal will do so far as it has a blood clotting system and a plasma kallikrein-kinin system. For example, human plasma and plasma of cattle and experimental animals such as cows, sheep, pigs, horses, goats, monkeys, dogs, cats, rabbits, guinea pigs, hamsters, rats or mice may be utilized. Preferaly, human plasma is used.

Such a plasma may be used in the form of a preparation manufactured by common methods. For example, a citric acid-added plasma prepared by collecting blood in the presence of citric acid followed by centrifuging may be used. Alternatively, freeze-dried plasma which is manufactured by conventional methods may be used. The animal plasma prepared as such may be used as It is or may be diluted, if desired, to control the reaction rate.

With reference to the FXII activator, any substance may be used so far as it exhibits an activating action for FXII such as, for example, glass, kaolin, celite, collagen, homocystine, sodium urate, cell components of platelets and other cells, fibronectin, elaidic acid, quercetin, rutin, sulfated glycolipids, proteoglycan, mucopolysaccharides, sodium stearate, dextran sulfate, amylose sulfate, carrageenin and proteases which activate FXII by a restricted decomposition. They may be used either solely or jointly and the concentration of such FXII activators may be suitably selected.

The mixing reaction of the animal plasma, FXII activator and tested substance is preferably carried out at 0°C to 4°C so that the intrinsic inhibitor hardly acts and, in addition, the progress of the reaction can be easily controlled. The pH during the reaction may be suitably selected but, when human plasa is used, it is preferred to carry out the reaction at pH 7.0 to 9.0. Further, in order to set at an optimum reaction condition, salts such as sodium chloride, metal ions such as zinc ion and other additives and auxiliary agents which are commonly used in this art may be added to the reaction system.

The reaction time may be suitably selected depending upon the amount of the animal plasma, the concentrations of the FXII activators and the tested substance and the pH of the reaction solution. etc. When, however, production of the FXIIa is saturated, it is no longer possible to precisely evaluate the action of the tested substance. Accordingly, it is preferred to set the time before the production of FXIIa is saturated, i.e. within a time where a clear positive relationship between the reaction time and the produced FXIIa (e.g., preferably in terms of the evaluation, within a time where a substantially linearly proportional relationship exists between them) is available.

With respect to a method for stopping the above-mentioned reaction, it is preferred to use a substance (which stops the production of FXII in the reaction such as an inhibitor for activation of FXII such as polybren) and an inhibitor (which specifically inhibits the plasma kallikrein, preferably a plasma kallikrein inibibitor which does not substantially affect FXIIa such as SBTI [soy bean trypsin inhibitor; a trypsin inhibitor prepared from soy bean]) whereby the produced FXIIa can be quantitatively determined utilizing an enzymatic activity of FXIIa as a target using a substrate to FXIIa. The concentrations of those inhibitors may be suitably selected within such an extent that they do not substantially affect the FXIIa activity.

In the plasma kallikrein-kinin system, a feedback mechanism wherein the production of FXIIa is promoted by the produced plasma kallikrein has been known but the activity of a tested substance to the production of FXIIa by a pure FXIIa activation reaction which does not proceed through said feedback mechanism may be determined by the present invention as well. In that case, (a) the plasma in which plasma prekallikrein is in a substantially deficient state prepared by such a means that an inhibitor which is specific to the plasma kallikrein such as the above-mentioned SBTI is previously added to the reaction system prior to the activation of FXII or (b) the prekallikrein-deficient plasmaper se or that which is made in a substantially prekallikrein-deficient state by such a means that the plasma prekallikrein is removed from or inactivated in the normal plasma is used as the animal plasma whereby the present invention can be carried out.

The quantitative determination of the produced FXIIa can be carried out by conventional methods. For example, a method in which the measurement is conducted using a substrate to FXIIa utilizing an enzymatic activity of FXIIa may be used. A method using natural substrates such as plasma prekallikrein, blood coagulation factor XI or plasmin; coloring synthetic substrates such as D-Pro-Phe-Arg-pNA and D-Leu-Gly-Arg-pNA; or fluorescent synthetic substrates such as Boc-Glu(OBz)-Gly-Arg-MCA or Boc-Gln-Gly-Arg-MCA is often and commonly used because of its easiness and simplicity. Besides the above-mentioned measuring methods using the substrates, immunological measuring methods such as radioimunoassay (RIA), enzyme immunoassay (EIA). etc.. a quantitative analytic method utilizing chromatography and the like may be used.

### (Examples)

The present invention will be further illustrated by way of the following examples.

To a solution comprising diluted human plasma, a certain amount of a substance to be tested (an analyte) and 25mM Tris hydrochloride buffer (pH: 8) containing 90 mM NaCl dextran sulfate was added to make its final concentration 2 micrograms/liter. The mixture was incubated in ice water for 15 minutes and then polyburene and SBTI were added thereto to make their final concentrations 200 micrograms/ml and 40 micrograms/ml, respectively. The reaction solution was incubated in a Tris hydrochloride buffer(pH: 8) together with 1 mM of D-Pro-Phe-Arg-pNA at 30° C for 30 minutes, the reaction was stopped by adding citric acid thereto, the mixture was centrifuged at 3,000 rpm for ten minutes and the absorbance (which corresponds to the produced amount of FXIIa) of the resulting supernatant liquid at 405 nm was measured.

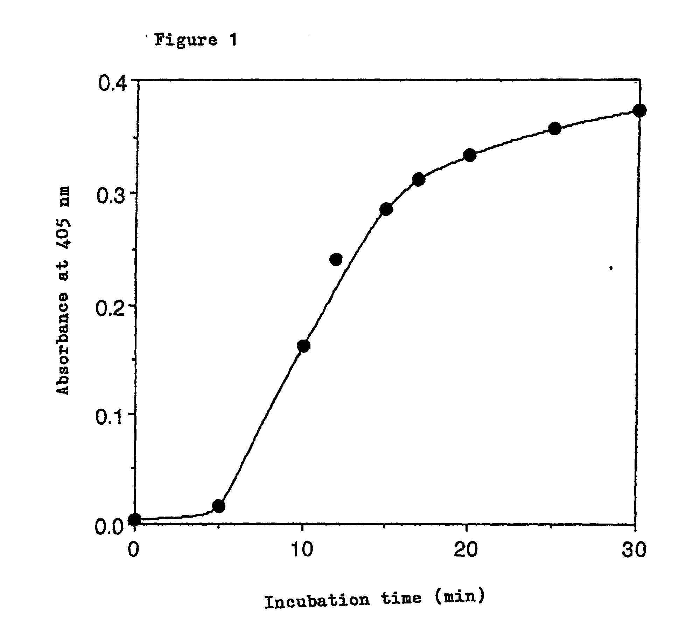

Fig. 1 shows the changes in the absorbances when the incubating time in the above reaction is changed. In Fig. 1, the abscissa shows the incubating time (minutes) at 0°C while the ordinate shows the absorbance at 405 nm. In Fig. 1. FXIIa is produced in almost linear increase with lapse of time until the incubating time becomes about 17 minutes and, therefore, it is preferred that the incubating time is to be set between 0 to 17 minutes.

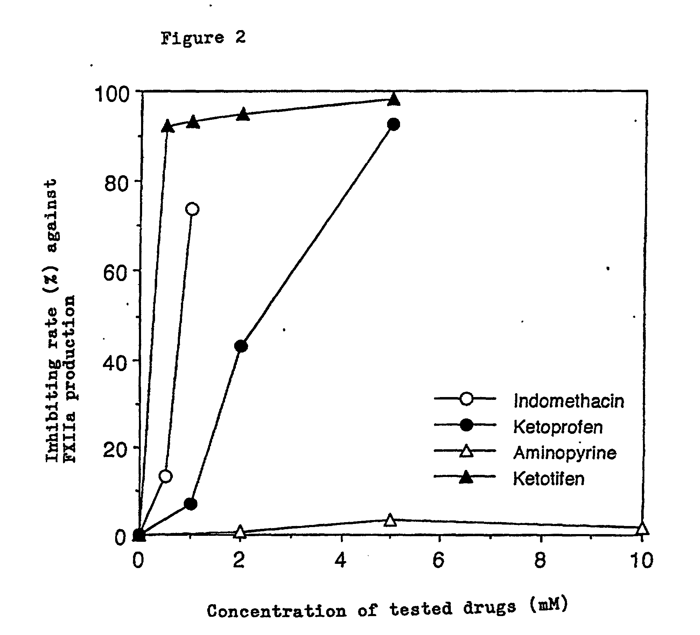

Fig. 2 shows the result of the measurement of the inhibiting activity of four test drugs which are used as analgesic or antiallergic agents (indomethacin. ketoprofen, aminopyrine and ketotifen) to the FXIIa production in accordance with the measurring method of the present invention. In Fig. 2, the abscissa shows the concentration (mM) of the drugs tested while the ordinate shows the inhibiting rate (%) to the FXIIa production.

FXIIa plays a very important role in the initiation stage of intrinsic blood clotting system, fibrinolysis system and plasma kallikrein-kinin system. Accordingly, the method of the present invention in which the activity of a tested substance having an action of inhibiting or promoting the FXIIa production can be measured is very highly useful in screening the drugs related to blood clotting system and plasma kallikrein-kinin system such as blood pressure controlling agents, antinflammatory agents, analgesics. antiallergic agents, etc.

Further in accordance with the measuring method of the present Invention, the feedback machanism of the plasma kallikrein for the FXIIa production is secured whereby it is possible to screen the drugs which solely relate to the activating stage of FXII which is the initiation stage of the reaction in the intrinsic blood clotting system, fibrinolysis system, plasma kallikrein-kinin system, etc. Consequently, screening of the drugs based upon a clear and specific action mechanism is now possible and the present invention has a significant merit on the development of various new drugs.

### Brief Explanation of the Drawings:

Fig. 1 is a graph showing the changes in the absorbance in various incubation time *in* the measuring method of the present invention.

Fig. 2 is a graph showing the result in the measurement of the inhibiting activity of some tested substances to the FXIIa production using the activity measuring method of the present invention.

Fig. 3 is a scheme for illustrating the regulation mechanism in vivo wherein FXIIa is participated.

## Claims

1. A method of measuring the activity of a test substance, characterized by the following steps:
i) initiating an activation reaction of blood coagulation factor XII by adding a blood coagulation factor XII activator to animal plasma in the presence of the test substance,
ii) stopping the reaction, and
iii) quantitatively determining the blood coagulation factor XII in its active form, produced in the above reaction.

2. A method according to claim 1 in which the production of the blood coagulation factor XII in an active form is stopped before this production becomes saturated.

3. A method according to claims 1 or 2 in which the blood coagulation factor XII in its active form is determined by using a substrate.

4. A method according to claim 3 in which the substrate is a synthetic substrate.

5. A method according to any one of claims 1 to 4 in which the blood coagulation factor XII is prevented from being produced in its active form via the plasma kallikrein-kinin feedback mechanism by either:
i) the addition of an inhibitor specific to plasma kallikrein prior to the activation of blood coagulation factor XII, or
ii) the removal of or deactivation of the plasma prekallikrein in the normal plasma, or
iii) using a prekallikrein-deficient plasma.

6. A method according to claim 5 in which the inhibitor used in (i) is soy bean trypsin inhibitor.

## Patentansprüche

1. Verfahren zur Messung der Aktivität einer getesteten Substanz, gekennzeichnet durch die folgenden Schritte:
(i) Initiierung einer Aktivierungsreaktion von Blutkoagulationsfaktor XII durch Zugabe eines Blutkoagulationsfaktor XII-Aktivators zu Tierplasma in Gegenwart der Testsubstanz,
(ii) Beenden der Reaktion und
(iii) quantitative Bestimmung des Blutkoagulationsfaktors XII in seiner aktiven Form, gebildet in der obigen Reaktion.

2. Verfahren gemäss Anspruch 1, worin die Bildung des Blutkoagulationsfaktors XII in einer aktiven Form beendet wird, bevor diese Bildung gesättigt ist.

3. Verfahren gemäss Anspruch 1 oder 2, worin der Blutkoagulationsfaktor XII in seiner aktiven Form durch Verwendung eines Substrats bestimmt wird.

4. Verfahren gemäss Anspruch 3, worin das Substrat ein synthetisches Substrat ist.

5. Verfahren gemäss mindestens einem der Ansprüche 1 bis 4, worin verhindert wird, dass der Blutkoagulationsfaktor XII in seiner aktiven Form über den Plasmakallikrein-Kinin-Feedback-Mechanismus gebildet wird, durch entweder:
(i) Zugabe eines Inhibitors, der spezifisch auf Plasmakallikrein ist, vor der Aktivierung des Blutkoagulationsfaktors XII, oder
(ii) Entfernung oder Deaktivierung des Plasmapräkallikreins im normalen Plasma, oder
(iii) Verwendung von Plasma, worin es an Präkallikrein mangelt.

6. Verfahren gemäss Anspruch 5, worin der verwendete Inhibitor in (i) Sojabohnen-Trypsin-Inhibitor ist.

## Revendications

1. Méthode de mesure de l'activité d'une substance à tester, caractérisée par les étapes suivantes:
(i) on amorce une réaction d'activation du facteur XII de la coagulation sanguine par addition d'un activateur du facteur XII de la coagulation sanguine à un plasma animal en présence de la substance à tester,
(ii) on stoppe la réaction et
(iii) on détermine d'une manière quantitative le facteur XII de la coagulation sanguine sous sa forme active, produit dans la réaction ci-dessus.

2. Méthode selon la revendication 1, dans laquelle la production du facteur XII de la coagulation sanguine sous une forme active est stoppée avant que cette production ne soit saturée.

3. Méthode selon la revendication 1 ou 2, dans laquelle on détermine le facteur XII de la coagulation sanguine sous sa forme active en utilisant un substrat.

4. Méthode selon la revendication 3, dans laquelle le substrat est un substrat synthétique.

5. Méthode selon l'une quelconque des revendications 1 à 4, dans laquelle on évite que le facteur XII de la coagulation sanguine ne soit produit sous sa forme active par l'intermédiaire du mécanisme de rétroaction kallicréine-kinine du plasma par:
i) soit addition d'un inhibiteur spécifique de la kallicréine du plasma avant l'activation du facteur XII de la coagulation sanguine,
ii) soit par élimination ou par désactivation de la prékallicréine du plasma dans le plasma normal,
iii) soit par utilisation d'un plasma déficient en prékallicréine.

6. Méthode selon la revendication 5, dans laquelle l'inhibiteur utilisé dans l'étape (i) est l'inhibiteur de trypsine de soja.
